Europäisches Patentamt

European Patent Office

Office européen des brevets

(11) Publication number: **0 303 406**
**A2**

# (12) EUROPEAN PATENT APPLICATION

(21) Application number: 88307235.7

(22) Date of filing: 05.08.88

(51) Int. Cl.4: **G01N 33/547 , G01N 33/543 , G01N 33/538**

(30) Priority: 14.08.87 US 85543

(43) Date of publication of application:
15.02.89 Bulletin 89/07

(84) Designated Contracting States:
CH DE FR GB LI

(71) Applicant: Hewlett-Packard Company
3000 Hanover Street
Palo Alto California 94304(US)

(72) Inventor: Myerson, Joel
1320 Kopkins Street
Berkeley California 94702(US)
Inventor: Yim, Kalvin W.
919 Fallen Leaf Way
Redwood City California 94062(US)

(74) Representative: Williams, John Francis et al
J.F. Williams & Co 34 Tavistock Street
London WC2E 7PB(GB)

(54) **An immunoaffinity substrate.**

(57) Novel affinity columns are provided by linking glycoproteins through the carbohydrate residue to supports. Oxidative cleavage of a sugar residue provides an aldehyde, which is condensed with an active amino functionality on the surface (and subsequent reduction if necessary) to provide for affinity columns where binding sites are substantially unaffected by the chemistry and are oriented to optimize binding to the complementary binding member.

EP 0 303 406 A2

# AN IMMUNOAFFINITY SUBSTRATE

## Technical Field

Packings for immunoaffinity chromatography are provided employing stable bridges between the support and the binding protein for orienting the binding sites of the immunoglobulin to enhance binding availability.

## Background

Any interest in biological processes, both natural and those involving external manipulation such as recombinant techniques, almost invariably involves purification of a protein from a complex protein mixture. A large number of techniques have been developed to separate components of a complex mixture as a result of different characteristics. These techniques include electrophoresis, thin layer chromatography, high performance liquid chromatography (HPLC), medium pressure liquid chromatography (MPLC), gravity flow chromatography, gradient density separations, or the like. Of particular interest are those techniques which allow for specific selection of one or a few protein components of a mixture, such as components having a specific epitopic site. These techniques inherently involve a receptor, such as an antibody or other specific binding protein, for specifically binding to a unique epitope, so as to retain those proteins having the epitope on a support in a column, while the other components of the mixture pass through with the void volume.

In order to ensure the substantial purity of the product of interest, relatively high affinity receptors are employed. Subsequently, to break the complex and release the product of interest, relatively rigorous conditions are employed. It is important that the affinity packing be able to withstand these conditions without degradation or release of the binding receptor. Release of the binding receptor would serve to contaminate the product, as well as limit the repetitive use of the chromatographic packing. Since it is desirable to be able to reuse the packing repeatedly, there is an interest in developing methods which retain the receptor bound to the column during the steps of adding the sample to the column, eluting, and displacing the product from the column.

There is a further interest in being able to orient the receptor, so as to maximize the available binding sites and minimize steric interference at the binding site receptor. Various techniques which have been employed suffer from numerous dificiencies in providing for unpredictable results, inactivation of the binding site, instability and leakage during chromatography, as well as other deficiencies.

## Relevant Literature

Phillips et al. describe the use of protein A-antibody interaction for use in preparation of chromatographic columns. Bauman et al., J. Histochem. Cytochem. (1981) 29:227-237 reports the use of fluorosceinsemicarbazide for coupling to periodate oxidized RNA. Phillips et al., J. of Chromatogrpahy (1985) 327:213-219 describes the use of protein A coated glass beads in immunoaffinity chromatography. Quash et al., J. Immunological Methods (1978) 22:165-174 describes the preparation of latex particles with covalently bonded polyamines, IgG and measles agglutinins. Periodate is used to oxidize carbohydrate residues for reductive amination. Phillips, L. C. Magazine (1985) 3:962-972 describes high performance immunoaffinity chromatography in a review article. Tijssen, Laboratory Techniques in Biochemistry and Molecular Biology, eds. Burdon and Knippenberg, Chapter 11, Preparation of Enzyme-antibody or Other Enzyme-macromolecule Conjugates, Elsevier, pp 221-241, provides a review of macromolecule conjugation.

## SUMMARY OF THE INVENTION

Glycosylated proteins are bound to chromatographic packings through stable linkages resulting from the reaction of aldehydes, produced by the glycol cleavage of the carbohydrate portion of a glycoprotein, through a primary amino functionality.

## DESCRIPTION OF THE SPECIFIC EMBODIMENTS

Affinity chromatography substrates are provided, where a glycoprotein, particularly a receptor, is oxidized by glycol cleavage to produce an aldehyde, usually a dialdehyde, for linking to the substrate. The resulting aldehydes are reacted with a primary amino functionality, whether as an amino group or as a portion of a hydrazine derivative such as thiosemicarbazide, in the former case desirably under reductive amination conditions. The amino functionality is linked to the support prior to or subsequent to the reaction with the aldehyde. The resulting supports are found to be stable under the rigorous conditions of affinity chromatography, allowing for ease of elution of contaminating components and release of the desired product, without significant degradation of the support or contamination of the product.

As receptors, of particular interest are immunoglobulins. The immunoglobulins may be IgA, IgD, IgE, IgG, and IgM or any subtype thereof. The immunoglobulins may be from any vertebrate source, such as mouse, rabbit, human, chicken, rat, or the like. The immunoglobulin may be monoclonal or polyclonal or mixtures thereof. The whole immunoglobulin may be used or fragments thereof, provided that the fragment retains the carbohydrate residue. Since all of the immunoglobulins are glycoproteins, they will have available sugars at the constant region which can be oxidized and used to link to the substrate, while orienting the immunoglobulin to provide available active sites. In this manner, the number of specific immunoglobulin binding sites per unit of surface area available for binding can be substantially enhanced over other techniques. Instead of a receptor a glycoprotein antigen may be employed in the same manner for purification of the complementary receptor.

A wide variety of solid supports may be employed, such as silica or controlled-pore glass (CPG) beads, as the most common packings. Two types of available silica include Nugel-P (Diagnostic Specialties, New Jersey), an irregular-shaped packing material having a series of different reactive side chains derivatized onto the surface of the silica, and epoxy-derivatized silica (Beckman Instruments, CA). The chemistry of glass beads is similar to that of silica. Particles used in chromatography are typically porous and have an average diameter of 2-100µ, more typically 3-75µ, and generally about 10-50µ. The pressure required to carry out chromatography varies with the particle size, with smaller particles requiring higher pressures.

Reactive groups which initially may be present on such supports include amino, epoxy, thiol, aminoaryl, carboxyl, carbonyldiimidazole, and N-hydroxysuccinimide ester. For the most part, an amino group on the surface will be present. However, where other than an amino group is involved, a linking amine, e.g. alkylene diamine, may be employed to react with the active functionality to form a stable bond. For example, the alkylene diamine may react with an epoxy to form an hydroxy diamine, or with a carboxyl, carbonyldiimidazole, or N-hydroxysuccinimide ester to form the aminoalkylamide. The amino groups may then be used to react with the glycol cleaved glycoprotein to provide for the affinity support. Alternatively, the linking amine compound or amino-linker may be first reacted with the oxidized glycoprotein, followed by reaction of the product with the activated particle. Various linking arms may be employed with due consideration for the functionalities on the oxidized glycoprotein. Linking arms may include 2-maleimidoethylamine, which may react with a mercapto group; o-hydroxyphenethylamine, which may react with a diazo functionality; 3-mercaptopropylamine, which may react with an activated olefin; and p-carboxyphenylhexylamine, which may react with an amine. The binding to the support need not be covalent. For example, biotinylated amine may be reacted with the aldehyde and the support may be functionalized with streptavidin or avidin. The strong binding affinity and excess avidin binding sites will ensure the maintenance of the glycoprotein on the support.

The formation of the aldehyde from the glycoprotein can employ conventional conditions. Periodate oxidation is conveniently employed in an aqueous medium at a mildly to moderately acidic pH, generally in the range from about 2 to 7. Low temperatures may be employed, generally in the range from -10 to 20° C, preferably from about 0 to 10° C, in the substantial absence of light. After sufficient time for the reaction to occur, usually about 30 min. and not more than about 6 h, excess periodate may be destroyed employing glycerol or ethylene glycol. Other oxidants which may be used to cleave vicinal glycols include lead tetraacetate; osmium tetroxide, particularly in conjuction with hydrogen peroxide; permanganate; etc. The oxidized glycoprotein may be dialyzed or gel filtered to remove undesired contaminants, particularly low molecular weight aldehydes.

The aldehyde may be reacted directly with the amino group in the presence of a reducing agent, at a pH from about 5 to 9, the pH being selected for the particular reducing agent. Reducing agents of particular interest are the borohydrides, for example, sodium borohydride, sodium cyanoborohydride (pH 5-7) or the like. Other metal hydrides may also find use, e.g., aluminum hydrides, although non-aqueous conditions will be required for some reducing agents, as is known in the art. Various buffers may be used, conveniently acetate buffer, particularly at about 0.05 to 0.2 M. Advantageously, with sodium cyanoborohydride, the pH is about 4.5 for the oxidation step and about 6 for the reductive amination step (acetate buffers can be used for both steps). The temperature will generally be below about 10°C, generally in the range of about 0 to 10°C. Reaction times will usually be at least about 0.5 h and not more than about 24 h. The particles may then be washed free of any non-conjugated protein.

Instead of a direct reaction between the amino group and the aldehyde, the amino may be further functionalized to form the thiosemicarbazide or other amino or hydrazine derivative. For example, commercially available amino-silica can be reacted in a chlorinated hydrocarbon solvent with toluene diisothiocyanate in the presence of a tertiary amine. Reaction of the resulting intermediate with anhydrous hydrazine provides the desired thiosemicarbazide-activated silica. Alternatively, commercially available aminosilica can be slurried in chloroform and reacted with thiophosgene. The intermediate is again reacted with anhydrous hydrazine followed by washing in an alcohol solvent to provide the desired thiosemicarbazide silica. Other stabilized derivatives of ammonia known to react to form stable derivatives with aldehydes, such as hydroxylamine, hydrazine, and phenylhydrazine, can be used in place of a semicarbazide linking group.

The affinity column may then be prepared by conventional means. Commonly, glass or stainless steel columns are employed. Pumped slurry packers or gas-activated pressure packers may be employed, frequently using aqueous packing solvents, generally buffered with 0.01 M phosphate or 0.1 M Tris. Depending on the size of the particles being used (and to some extent the personal preference of the user), the packing procedure can be carried out using a slurry and high-pressure, low-pressure, or any pressure in between. The high-pressure limit for silica is about 10,000 psi, but these pressures cannot be used for glass columns. The low-pressure limit is a few psi, as when packing occurs using gravity flow. Silica packing materials having diameters greater than about 10μ can also readily be dry packed.

Generally the columns are operated at flow rates of about 0.5-1.5 ml/min. The sample is applied to the column and allowed to flow through the column, where the specific protein becomes bound to the particles and the other components pass through the column and exit from the column. The column may then be washed with a wide variety of solvents to remove any residual contaminating materials, followed by elution, where the receptor-protein complexes are broken and the protein is removed from the column.

Various eluting solvents may be employed, usually either acidic, chaotropic, or denaturing. Acidic buffers include 0.1 M glycine, pH 1.5; 0.1 M Trisglycine, pH 1.0; 0.33 M citric acid, pH 2.0; 0.9% NaCl-0.1 M sodium acetate- 0.1 M HCl, pH 1.0; 20% formic acid, pH 1.8; 1 M acetic acid, pH 2.0; etc. Chaotropic agents include 2.5 M sodium thiocyanate, 2.5 M sodium iodide, 2.5 M polyvinylpyrrolidone, 6-8 M NaCl, etc. Denaturing agents include 8 M urea, and 6 M guanidine-HCl, pH 3.0. Other agents include 50% ethylene glycol, pH 11 and 10% dioxane. These agents are passed through a column and the exiting solvent monitored following the release of protein in the eluting solvent.

After the column has been freed of all or substantially all of the non-covalently bound protein, the column may then be washed to remove the eluting solvent and be ready for repetitive use. The subject columns are found to be highly stable, so as to be repetitively reused, as well as providing for minimal loss of the binding protein. In this manner, the capacity of the column is maintained and there is minimal contamination of the desired product. In addition, a high ratio of available binding sites to surface is achieved, which enhances the efficiency of the column providing for a higher binding capacity for a given packing value.

The following examples are offered by way of illustration and not by way of limitation.


## EXPERIMENTAL


The following is a paradigmatic procedure, which may be used with a wide variety of packings, which are commercially available or may be prepared individually to provide for specific functional groups or linking arms on the support.

The antibody solution, whether monoclonal or polyclonal is dispersed in 0.1 M sodium acetate buffer. This can be achieved by passing the antibody solution through a gel filtration column which has been equilibrated in 0.1 M sodium acetate plus 0.15 M sodium chloride, pH 4.5 buffer, and using the same buffer as the mobile phase. To the buffered antibody solution is added 0.1 M sodium periodate to bring the final solution to 20 mM in sodium periodate. The solution is gently mixed at 4°C in the dark by end-over-end agita tion for 1 h. The sodium periodate and its by-products are then removed from the antibody solution by passing the solution through a second equilibrated gel filtration column employing the same buffer. A suitable amount of functionalized support, e.g. silica with an amino functional group attached to a spacer arm of 12 angstroms length is added to the antibody solution. The pH is adjusted to pH 6 by addition of sodium carbonate solution and the mixture is gently agitated in an end-over-end manner for 1 h at 4°C. Sufficient sodium cyanoborohydride (based on the amount of antibody present) is added to the mixture. For example, with an initial amount of 1 mg antibody, 2 mg sodium cyanoborohydride is added. After mixing the reaction mixture end-over-end overnight at 4°C the resulting mixture is packed into a column, and washed sequentially with 0.5 M sodium thiocyanate and phosphate-buffered-saline. The column is now ready for use in affinity chromatography. The procedure for attachment to a thiosemicarbazide silica is similar, except the pH is maintained at about 4.5 and the cyanoborohydride reduction is omitted.

The following examples demonstrate the application of the subject method in the preparation of affinity columns and their use for purification of proteins.

### 1) Purification of Biologicals from Culture Fluid.

Anti-tPA antibody (2.2 mg) (tPA-tissue plasminogen activator) is immobilized onto 0.38 g of thiosemicarbazide silica as described above. The capacity of this column is found to be 0.72 mg of tPA by an ELISA assay. Assuming one anti-tPA antibody molecule binds one tPA molecule (as found empirically) that translates to 1500 mg of anti-tPA will bind 630 mg of tPA theoretically. The theoretical capacity of the 2.2 mg anti-tPA column is 0.92 mg. Therefore the capacity of the 2.2 mg anti-tPA column is 78% of the theoretical capacity.

A tPA culture fluid which contained approximately 50 μg of tPA/ml and 100 fold of other proteins was employed. The culture fluid (10 ml) was passed through the 2.2 mg anti-tPA column. All of the contaminating proteins were washed off the column, while only tPA was retained on the column. As a result, about 0.5 mg of purified tPA was concentrated in a volume of 1.3 ml when eluted with 0.1 M acetic acid. The process required 28 min (the loading of 10 ml of sample at 5 ml per minute takes 20 min, and elution takes 8 min). The time can be further reduced by using a column of wider diameter. The purity of the product was ascertained by SDS-PAGE.

### 2) Purification of Antibodies

tPA (0.61 mg), a glycoprotein was immobilized onto 0.38 g of amino-silica (Separation Industries). The resulting column binds 0.36 mg of anti-tPA obtained from ascites fluid. The solvent loading buffer (solvent A) is 20 mM sodium phosphate, 0.15 M NaCl, 0.025% Tween 20, pH 6.8. The elution buffer (solvent B) is 0.1 M acetic acid, 0.15 M NaCl, 0.025% Tween 20. The solvent program is as follows: 0-2.5 min, 0% B; 2.5-3.5 min, 0-100% B; 3.5-7 min, 100% B. The anti-tPA came off as a sharp peak at about 4 min and 50 seconds and removal was complete at about 6 min. Albumin and transferrin, which constitute over 50% of the proteins in the ascites fluid, are not retained on the column and are washed off the column. A cation exchange chromatogram of the anti-tPA eluted off the column indicated the anti-tPA to be pure.

### 3) Purification of Polyclonal Antibodies.

Less than 10% of antibodies in a particular antiserum are reactive against the immunizing antigen. Immobilized antigen can be used to purify the antisera. Substantially pure antibody against tPA from polyclonal antisera was obtained by employing the procedure described above.

### 4) Purification of Rat Antibodies.

Mouse-(anti-rat immunoglobulin) was employed to prepare an affinity column for purification of rat antibodies. The column was prepared by employing support with 2 mg mouse-anti-rat antibodies. By employing the same procedures described above, substantially pure rat antibody was obtained.

5) Comparison of Various Chemistries.

To evaluate the overall recovery of binding capacity of various immobilization chemistries, four columns were prepared using four different chemistries:

1) 2.2 mg of anti-tPA was immobilized onto 1 gram of amino-silica as described above;

2) 2.2 mg of anti-tPA was immobilized onto 1 gram of amino-silica using water soluble carbodiimide (EDC) chemistry as described in a procedure provided by Separation Industries;

3) 2.2 mg of anti-tPA was immobilized onto 1 gram of hydroxyl-silica using the periodate chemistry as described in the procedure provided by Separation Industries. See S.K. Roy et al., J. Chrom. (1984) 303:225-228.

4) 2.2 mg of anti-tPA was immobilized onto 1 gram of Reacti-gel (CDI-activated TSK HW-65F) using the carbonyl diimidazole (CDI) chemistry as described in instructions provided by the vendor (Pierce Chemical Co.).

The capacity of the different methods are shown below relative to the capacity of the subject method. The purity of the peak material is ascertained by SDS-PAGE. The results are tabulated as follows.

| Column | Relative Capacity (Col. 1 equals 1.00) |
| --- | --- |
| 1 | 1.00 |
| 2 | 0.58 |
| 3 | 0.11 |
| 4 | 0.18 |

6) Realtime Monitoring of Tissue Culture

The subject column was used to monitor the production of recombinant protein products. The monitoring is important since life cycles of microorganisms are short and in order to optimize the product yield, realtime yield monitoring is essential. In view of the rapidity of the subject immunoaffinity assay, (8 min) the procedure described above can be used for monitoring the yield of a recombinant product such as tPA. It was found that one could readily monitor the yield to tPA during the course of the tPA production from a microorganism transformed with a functional tPA expression cassette.

The subject invention has a significant number of advantages. It can be used with all antibodies, since all immunoglobulins have a carbohydrate chain in the constant non-binding region of the immunoglobulin molecule. Immobilizing of the antibody employing the carbohydrate chain offers a number of advantages: 1) the antigen binding site is not involved in the immobilization to the support and therefore is available for antigen binding; 2) the affinity binding is not affected by the immobilization, so that the binding site is substantially preserved in its normal conformation; 3) the antigen binding region (Fab) is oriented outward (from the particle) and therefore optimum for antigen binding; 4) there is only one carbohydrate chain per heavy chain and so the attachment is reproducible. Therefore, the columns prepared by this method have higher capacity than columns prepared by conventional methods, can be readily prepared using safe chemistries, and can provide for reproducible capacities, so as to allow for semiquantitative or quantitative determinations.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

**Claims**

1. An immunoaffinity substrate comprising a glycoprotein having a binding site for an epitope or idiotype of interest bonded to a support characterised in that the glycosyl moiety is covalently bonded to the support by a linkage containing a group formally derivable from the reaction of an aldehyde and an amino group or a derivative of said group obtained by subsequent reduction thereof.

2. An immunoaffinity substrate according to claim 1, in which the amino group is a primary amino group.

3. An immunoaffinity substrate according to claim 1 or 2, in which the (amino) nitrogen atom of the linkage is bonded directly to the support material.

4. An immunoaffinity substrate according to claim 1 or 2, in which the (amino) nitrogen atom of the linkage is bonded indirectly to the support material through a linker arm.

5. An immunoaffinity substrate according to claim 4, in which the (amino) nitrogen atom of the linkage is part of a hydrazino radical.

6. An immunoaffinity substrate according to claim 4, in which the (amino) nitrogen atom of the linkage is part of a semicarbazone or thiosemicarbazone linkage.

7. An immunoaffinity substrate according to any of claims 1 to 6, in which the glycoprotein is bonded to the support by at least two linkages of the kind described.

8. An immunoaffinity substrate according to any of the preceding claims, having a support in the form of silica particles, controlled pore glass beads, or other particulate form.

9. An immunoaffinity column material according to any of the preceding claims.

10. A method of preparing an immunoaffinity substrate material which comprises oxidatively converting the carbohydrate portion of a glycoprotein having an epitope - or idiotype - binding site to form one or more aldehyde groups, reacting the aldehyde group(s) with a substance containing a reactive amino functionality ($-NH_2$ or $-NH-$), said substance being a support material or a linking compound which, after reaction with the aldehyde group(s), as reacted with a support material to form a covalent attachment thereto, and optionally reducing the radical (Schiffs base) produced by reaction of the aldehyde group(s) and the amino group(s).